# EUROPÄISCHE PATENTSCHRIFT

(11) **EP 2 154 083 B1**
(45) Veröffentlichungstag und Bekanntmachung des Hinweises auf die Patenterteilung: **07.09.2016**
(21) Anmeldenummer: 09009311.3
(22) Anmeldetag: 17.07.2009
(51) Int. Cl.: B65D 81/24, A01G 9/02, B65D 77/20, B65B 25/02, B65D 81/26, B29C 65/08, B29C 65/48, B29C 65/76

(54) **Behälter sowie Verfahren zur Herstellung einer Verbindung zwischen einem Unterteil und einem Oberteil eines Behälters**
Container and method for producing a connection between a lower part and upper part of a container
Récipient et procédé de fabrication d'une liaison entre une partie inférieure et une partie supérieure d'un récipient

(30) Priorität: 13.08.2008 DE 102008038902
(43) Veröffentlichungstag der Anmeldung: 17.02.2010
(73) Patentinhaber: Fritz Hark Orchideen GmbH & Co. KG, 59557 Lippstadt (DE)
(72) Erfinder: Kies, Frank, 58739 Wickede (DE); Lapornik, Karl-Heinz, 58739 Wickede (DE); Herrde, Mathias, 24558 Henstedt-Ulzburg (DE)
(74) Vertreter: Basfeld, Rainer

(56) Entgegenhaltungen:
- EP-A1- 0 282 773
- EP-A1- 0 376 413
- WO-A2-94/27868
- JP-A- 2001 301 864
- JP-A- 2003 143 986
- US-A- 3 780 926
- US-A- 4 124 141
- US-A- 4 582 239
- US-A- 4 693 390
- US-A- 4 704 254
- US-A- 5 129 531
- US-A1- 2004 011 794

## Beschreibung

Die vorliegende Erfindung betrifft einen Behälter für die Aufnahme von Gegenständen gemäß dem Oberbegriff des Anspruchs 1. Weiterhin betrifft die vorliegende Erfindung ein Verfahren zur Herstellung einer Verbindung zwischen einem Unterteil und einem Oberteil eines Behälters für die Aufnahme von Gegenständen gemäß dem Oberbegriff des Anspruchs 10.

Derartige Behälter sind bekannt und bestehen beispielsweise aus transparentem Kunststoff, Glas oder andere transparenten Materialien. Bei einer speziellen Anwendung werden in derartigen, zumindest teilweise transparenten Behältern Orchideen gezüchtet. Insbesondere werden diese Behälter für die Meristemkulturen von Orchideen verwendet. In derartige Behälter, die vor der Benutzung sterilisiert wurden, werden autoklavierte Nährböden abgefüllt. Auf diese Nährböden wiederum werden unter sterilen Laborbedingungen Pflanzenteile pikiert oder es werden die Nährböden mit anderen Zellkulturen beimpft. Nur eine sterile Umgebung sowie die sterilen Behälter können gewährleisten, dass die sehr empfindlichen Orchideen-Kulturen gedeihen können. Im Stand der Technik sind die Behälter für sterile Zellkulturen in der Regel nicht völlig dicht. Dabei weisen beispielsweise Oberteil und Unterteil umlaufende Ränder auf, die miteinander verbunden sind. Die Verbindung der Ränder miteinander ist dabei beispielsweise derartig, dass ein Gasaustausch zwischen Aufnahmeraum und Umgebung über den Verbindungsbereich der Ränder erfolgen kann.

Als nachteilig bei aus dem Stand der Technik bekannten undichten Behältern erweist sich, dass während der Lagerung oder des Transport des Behälters eine Infektion der Nährmedien durch Schädlinge wie Viren, Bakterien, Pilzsporen oder insbesondere Milben stattfinden kann. Die infizierten Behälter sind dann für die weitere Verwendung unbrauchbar. Schäden durch derartige Infektionen, insbesondere durch Milben, können den Laboren schwere wirtschaftliche Verluste zufügen.

Ein Behälter und ein Verfahren der eingangs genannten Art sind aus der EP 0 282 773 A1 bekannt. Der darin beschriebene Behälter weist ein Unterteil mit einer Öffnung und einem über die Öffnung zugänglichen Aufnahmeraum für Gegenstände auf. Auf dieses Unterteil ist ein Oberteil mittels Ultraschall aufgeschweißt, wobei die dabei entstehende Schweißnaht in umlaufenden, aufeinander liegenden Rändern des Oberteils und des Unterteils angeordnet ist. Zwischen der Ultraschweißnaht und der Öffnung des Aufnahmeraums ist in dem Rand des Unterteils eine umlaufende Nut vorgesehen, die als Mittel zum Lösen der Verbindung zwischen Oberteil und Unterteil dient. Außerhalb der Schweißnaht ragt der Rand des Oberteils über den Rand des Unterteils hinaus, so dass ein Benutzer zum Öffnen des Behälters an dem hervorstehenden Rand des Oberteils angreifen kann.

Aus der US 5,129,531 ist ein Behälter mit wiederverschließbarem Deckel bekannt. Der Deckel weist einen sich nach unten erstreckenden Flansch auf, der in eine Aussparung des vertikalen Behälterrandes einrasten kann. Alternativ kann der Flansch mit dem vertikalen Behälterrand über einen Schraubverschluss verbunden werden. Vor dem ersten Öffnen des Behälters umgibt ein Verschlussband den Deckel und den oberen Teil des Behälters. Dieses Verschlussband ist mit dem Flansch über eine Sollbruchstelle verbunden und kann an einem radial nach außen ragenden Vorsprung des vertikalen Behälterrandes durch Heißschrumpfen befestigt sein.

Aus der US 3,780,926 und der US 4,582,239 sind Ultraschallschweißgeräte mit einer Mehrzahl von Sonotroden bekannt.

Ein weiterer Behälter ist aus der deutschen Offenlegungsschrift DE 32 18 532 A1 bekannt. Auf die darin beschriebene Petrischale zur Züchtung von Bakterien und Kleinpilzen wird eine Folie als Deckel aufgeschweißt oder aufgeklebt, so dass eine dichte Verbindung zwischen Unterteil und Deckel entsteht. Der Deckel weist eine außerhalb des Schweißbereichs angeordnete Lasche auf, an der ein Benutzer so ziehen kann, dass der Deckel als Ganzes von dem Unterteil entfernt wird.

Als nachteilig bei diesem Stand der Technik ist anzusehen, dass der Deckel nach erstmaligem Abreißen nicht mehr so auf das Unterteil aufgebracht werden kann, dass er den Aufnahmeraum im Unterteil zumindest staubdicht und sicher von der Umwelt abschließt. Weiterhin kann der Deckel auch vor dem Aufschweißen das Unterteil nicht wirksam abdecken, so dass die Unterteile nicht in großer Stückzahl mit Nährboden befüllt werden können, bevor der Nährboden mit den zur Aufzucht vorgesehenen Pflanzenteilen versehen wird.

Das der vorliegenden Erfindung zugrunde liegende Problem ist die Schaffung eines Behälters der eingangs genannten Art, der besser für die Aufzucht von Pflanzen geeignet ist. Weiterhin soll ein Verfahren der eingangs genannten Art angegeben werden, mit dem sich Oberteil und Unterteil eines Behälters effektiver miteinander verbinden lassen.

Dies wird erfindungsgemäß hinsichtlich des Behälters durch einen Behälter der eingangs genannten Art mit den kennzeichnenden Merkmalen des Anspruchs 1 sowie hinsichtlich des Verfahrens durch ein Verfahren der eingangs genannten Art mit den kennzeichnenden Merkmalen des Anspruchs 10 erreicht. Die Unteransprüche betreffen bevorzugte Weiterbildungen der Erfindung.

Gemäß Anspruch 1 ist vorgesehen, dass die Mittel zum Lösen der Verbindung mindestens eine Aufreißlasche umfassen, die benachbart zu dem mindestens einen Sollbruchbereich an dem Unterteil oder an dem Oberteil angeordnet ist.

Die Verbindung von Unterteil und Oberteil kann gelöst werden, ohne dass das Oberteil von dem Unterteil abgenommen wird. Auch nach dem Abnehmen des Oberteils kann es wieder auf das Unterteil aufgesetzt werden und den Aufnahmeraum zumindest staubdicht und sicher von der Umwelt abschließen.

Es ist vorgesehen, dass sowohl das Oberteil, als auch das Unterteil einen zumindest teilweise umlaufenden Rand aufweisen, wobei die Ränder von Oberteil und Unterteil im verbundenen Zustand zumindest teilweise aneinander anliegen. Dabei ist die Verbindung zwischen Oberteil und Unterteil durch Verbindung der Ränder miteinander realisiert. Die beispielsweise aufeinander liegenden Ränder können umlaufend beispielsweise miteinander verschweißt werden.

Es kann vorgesehen sein, dass der mindestens eine Verbindungsbereich an einem der Ränder oder an beiden Rändern von Oberteil und Unterteil angeordnet ist. Beispielsweise kann der mindestens eine Verbindungsbereich zumindest teilweise mit einem der Ränder oder beiden Rändern umlaufen. Insbesondere kann dabei der mindestens eine Verbindungsbereich in Umlaufrichtung beziehungsweise Umfangsrichtung eine Länge von mehr als 20 cm, insbesondere von mehr als 40 cm, vorzugsweise eine Länge zwischen 50 cm und 55 cm aufweisen. Derartig große Verbindungsbereiche erlauben den keimfreien Verschluss von Behältern mit vergleichsweise großer Grundfläche.

Die Verbindung zwischen Oberteil und Unterteil kann beispielsweise als Schweißverbindung, insbesondere als Ultraschall- oder Laserschweißverbindung, als Heißprägeverbindung oder als Klebeverbindung ausgebildet sein. Derartige Verbindungen erlauben mit einfachen Mitteln ein keimfreies Verschließen des Aufnahmeraums. Dadurch wird verhindert, dass durch den Verbindungsbereich Schädlinge in den Aufnahmeraum eindringen.

Es besteht die Möglichkeit, dass das Oberteil und/oder das Unterteil als Kunststoffteile, insbesondere Spritzgussteile oder Tiefziehteile, oder Teile aus anderen transparenten Materialien ausgebildet sind. Vorzugsweise kann vorgesehen sein, dass das Oberteil und/oder das Unterteil zumindest teilweise oder abschnittsweise antimikrobiell ausgebildet sind oder antimikrobiell wirken. Durch eine zumindest teilweise oder abschnittsweise antimikrobielle Ausbildung oder antimikrobielle Wirkung des Oberteils und/oder des Unterteils des Behälters wird ein zusätzlicher Schutz vor Schädlingsbefall erreicht.

Die Mittel zum Lösen der Verbindung zwischen Oberteil und Unterteil umfassen einen Sollbruchbereich an dem Unterteil oder an dem Oberteil, der zwischen der Öffnung und dem mindestens einen Verbindungsbereich angeordnet ist. Dabei kann der Sollbruchbereich mindestens eine, vorzugsweise zwei, zumindest teilweise umlaufende Nuten umfassen. Auf diese Weise lässt sich einfach und zuverlässig eine gezielte Schwächung erreichen, die ein Lösen der Verbindung gewährleisten kann.

Weiterhin umfassen die Mittel zum Lösen der Verbindung zwischen Oberteil und Unterteil mindestens eine Aufreißlasche, die benachbart zu dem mindestens einen Sollbruchbereich, insbesondere zwischen den beiden Nuten, an dem Unterteil oder an dem Oberteil angeordnet ist. Eine derartige Aufreißlasche stellt ein einfaches und sehr funktionelles Mittel zum Lösen der Verbindung dar.

Dabei kann die mindestens eine Aufreißlasche senkrecht von dem Unterteil oder dem Oberteil wegragen und insbesondere im Querschnitt winkelförmig, wie beispielsweise L-förmig oder, V-förmig ausgebildet ist. Dadurch wird ein punktförmiges Aufreißen einer SollBruch-Linie ermöglicht.

Es kann weiterhin vorgesehen sein, dass das Oberteil und/oder das Unterteil eine Öffnung aufweisen, die mit einem Filter versehen ist oder versehen werden kann. Durch diese Öffnung kann ein Gasaustausch des Aufnahmeraums mit der Umgebung stattfinden. Der Filter kann so gestaltet sein, dass durch ihn zwar ein Gasaustausch stattfinden kann, nicht jedoch Schädlinge eindringen können. Unter Umständen kann der Filter auch antimikrobiell ausgebildet sein.

Die Öffnung kann beispielsweise in Gebrauchsstellung beabstandet von dem oberen Rand des Unterteils in einer der Seitenwände angeordnet sein. Dadurch kann gewährleistet werden, dass durch die Öffnung schädliche Gase wie beispielsweise CO₂ aus dem Behälter austreten können.

Ein erfindungsgemäßer Behälter ist insbesondere für die Aufnahme und/oder das Aufziehen von Pflanzen, insbesondere Orchideen, oder für die Aufnahme von Zell-, Pilz-, Viren- oder Bakterienkulturen im Bereich der Humanbiologie, der Veterinärbiologie oder der Pflanzenvermehrung geeignet.

Anspruch 10 sieht vor, dass für das Verschweißen eine Ultraschall-Sonotrode verwendet wird, die eine Mehrzahl von Segmenten aufweist, die zumindest abschnittsweise voneinander beabstandet sind, wobei die Breite der Segmente sich in Richtung auf den Schweißbereich vergrößert. Eine derartige Ultraschall-Sonotrode ermöglicht auch das Verschweißen von Oberteilen und Unterteilen mit langen Verbindungsbereichen in einem einzigen Arbeitsgang, so dass die Verbindung kostengünstig realisiert werden kann.

Weitere Merkmale und Vorteile der vorliegenden Erfindung werden deutlich anhand der nachfolgenden Beschreibung bevorzugter Ausführungsbeispiele unter Bezugnahme auf die beiliegenden Abbildungen. Darin zeigen
- Fig. 1: eine perspektivische Ansicht von unten eines Oberteils einer ersten Ausführungsform eines erfindungsgemäßen Behälters;
- Fig. 2: eine perspektivische Ansicht von oben eines Unterteils einer ersten Ausführungsform eines erfindungsgemäßen Behälters;
- Fig. 3: eine stirnseitige Ansicht des Unterteils gemäß Fig. 2;
- Fig. 4: eine dem in Fig. 3 mit IV bezeichneten Abschnitt entsprechende Detailansicht des Unterteils gemäß Fig. 2;
- Fig. 5: eine Schnittansicht eines Teils der miteinander verbundenen Ränder von Oberteil und Unterteil der ersten Ausführungsform;
- Fig. 6: eine Seitenansicht eines Unterteils einer zweiten Ausführungsform eines erfindungsgemäßen Behälters;
- Fig. 7: eine Draufsicht auf das Unterteil gemäß Fig. 6;
- Fig. 8: einen Schnitt durch zwei ineinander gestapelte Unterteile gemäß Fig. 6;
- Fig. 9: eine Detailansicht gemäß dem Pfeil IX in Fig. 8;
- Fig. 10: eine Detailansicht gemäß dem Pfeil X in Fig. 9;
- Fig. 11: eine Schnittansicht gemäß den Pfeilen XI - XI in Fig. 7;
- Fig. 12: eine Schnittansicht gemäß den Pfeilen XII - XII in Fig. 7;
- Fig. 13: eine perspektivische Ansicht eines Oberteils einer zweiten Ausführungsform eines erfindungsgemäßen Behälters;
- Fig. 14: eine Detailansicht gemäß dem Pfeil XIV in Fig. 13;
- Fig. 15: eine Detailansicht gemäß dem Pfeil XV in Fig. 13;
- Fig. 16: eine Draufsicht auf das Oberteil gemäß Fig. 13;
- Fig. 17: eine Schnittansicht gemäß den Pfeilen XVII - XVII in Fig. 16;
- Fig. 18: eine Schnittansicht gemäß den Pfeilen XVIII - XVIII in Fig. 16;
- Fig. 19: eine schematische Schnittansicht gemäß den Pfeilen XIX-XIX in Fig. 16, wobei zusätzlich zur Verdeutlichung ein zweites Oberteil abgebildet ist, das in das aus Fig. 16 ersichtliche Oberteil eingesetzt ist;
- Fig. 20: eine perspektivische Ansicht einer Sonotrode zur Durchführung des erfindungsgemäßen Verfahrens;
- Fig. 21: eine perspektivische Untenansicht eines Unterteils gemäß Fig. 6;
- Fig. 22: eine Detailansicht gemäß dem Pfeil XXII in Fig. 21.

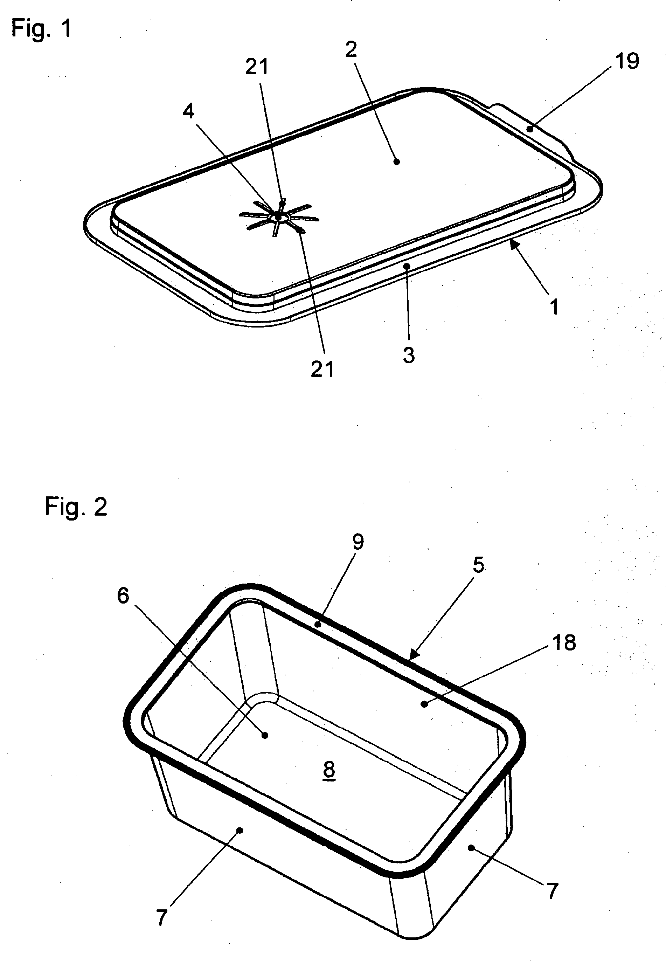

Aus Fig. 1 ist ein als Deckel ausgebildetes Oberteil 1 eines erfindungsgemäßen Behälters ersichtlich. Das Oberteil 1 besteht aus Kunststoff und ist beispielsweise ein Spritzgussteil. Das Oberteil 1 weist eine Deckelfläche 2 und einen diese umgebenden Rand 3 auf, der sich parallel zu der Deckelfläche 2 erstreckt.

In der Deckelfläche 2 ist eine Öffnung 4 vorgesehen, die im Gebrauch mit einem Filter (nicht abgebildet) verschlossen werden kann. Die Verbindung zwischen dem Filter und dem Oberteil 1 kann dabei als Schweißverbindung, insbesondere als Ultraschall- oder Laserschweißverbindung, als Heißprägeverbindung oder als Klebeverbindung ausgeführt sein. Weiterhin kommt auch eine IML-Verbindung (In Mould Labeling) in Betracht. Dabei wird der Filter direkt in das Spritzgusswerkzeug zur Herstellung des Oberteils 1 eingelegt und mit dem Spritzgussteil vergossen.

Der Filter kann als Membran ausgebildet sein und insbesondere durch Strecken einer einlagigen Schicht hergestellt werden.

Die Öffnung 4 ist von sternförmig angeordneten Vorsprüngen 21 umgeben. Diese Vorsprünge 21 können verhindern, dass ein Blatt einer in dem Behälter gezüchteten Pflanze flächig an der Öffnung 4 anliegt und diese dadurch verschließt. Die Vorsprünge 21 enden mit Abstand vor dem Rand der Öffnung 4. Dadurch entsteht eine Auflagefläche für einen Gegenstempel bei dem Aufpressen des Filters von außen auf das Oberteil 1.

An dem Rand 3 ist eine als Angriffmittel dienende Handhabe 19 vorgesehen, die mit einer Pinzette gegriffen werden kann. Durch dieses Angriffmittel und die dadurch ermöglichte Benutzung einer Pinzette wird verhindert, dass beim Aufsetzen des Oberteils 1 vor dem Verschweißen Randbereiche des im Nachfolgenden näher beschriebenen Unterteils 5 und/oder des Oberteils 1 durch Handberührung infiziert werden. Weiterhin kann die Handhabe 19 verhindern, dass der Daumen eines Benutzers beim Öffnen des Deckels in den Sterilbereich eindringt.

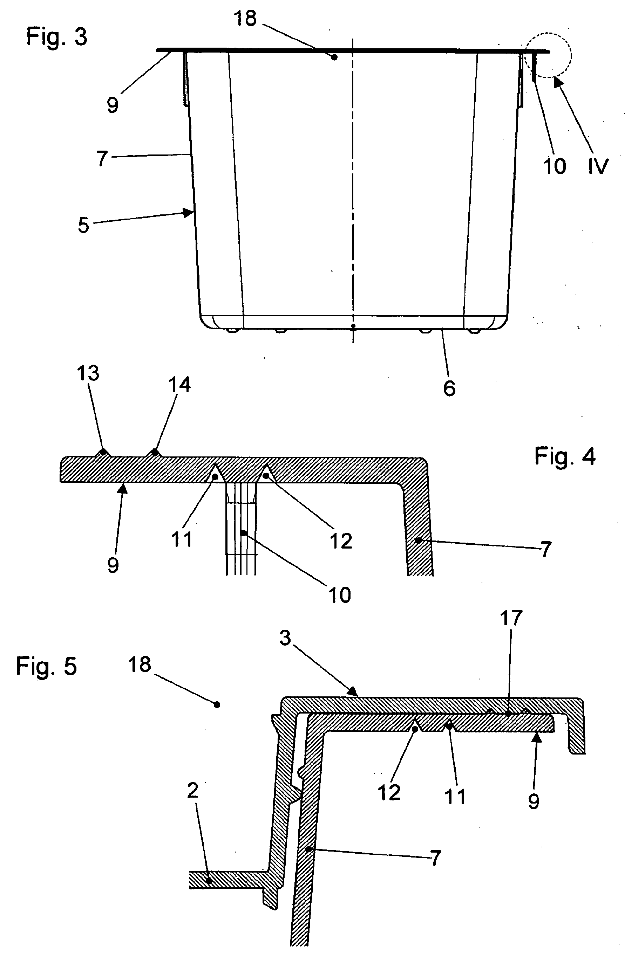

Fig. 2 und Fig. 3 zeigen das Unterteil 5 eines erfindungsgemäßen Behälters. Das Unterteil 5 besteht aus Kunststoff und ist beispielsweise ein Spritzgussteil. Das Unterteil 5 umfasst einen Boden 6 und Seitenwände 7, die einen Aufnahmeraum 8 umgeben. An dem oberen Ende der Seitenwände 7 ist ein umlaufender Rand 9 angeordnet, der sich parallel zu dem Boden 6 erstreckt. Das Unterteil 5 ist oben offen, so dass eine Öffnung 18 zur Einbringung von Gegenständen gebildet wird.

Der Rand 9 des Unterteils 5 ist aus Fig. 4 detailliert ersichtlich. Er weist eine Aufreißlasche 10 auf, die zwischen zwei umlaufenden Nuten 11, 12 angeordnet ist. Die mindestens eine Aufreißlasche 10 ragt senkrecht von dem Unterteil 5 weg und ist insbesondere im Querschnitt winkelförmig, wie beispielsweise L-förmig oder V-förmig ausgebildet.

Die umlaufenden Nuten 11, 12 erstrecken sich fast durch die gesamte Dicke des Randes 9 und schwächen den Rand 9 derart, dass der Benutzer durch Abbiegen und anschließendes Ziehen der Aufreißlasche 10 in Längsrichtung des Randes 9 diesen in zwei voneinander getrennte Abschnitte aufreißen kann. Die umlaufenden Nuten bilden also einen Sollbruchbereich.

Weiterhin weist der Rand 9 des Unterteils 5 zwei sich nach oben erstreckende Vorsprünge 13, 14 auf, die umlaufende sind. Die Vorsprünge 13, 14 sind auf der von den Seitenwänden 7 abgewandten Seite der Aufreißlasche 10 angeordnet. Die Vorsprünge 13, 14 dienen bei der im Nachfolgenden noch näher beschriebenen Ultraschallverschweißung als Energierichtgeber. Im Bereich der Vorsprünge 13, 14 kann die dichte bezeihungsweise keimfreie Verbindung zwischen dem Rand 3 des Oberteils und dem Rand 9 des Unterteils 5 realisiert werden. Mit dem Bezugszeichen 17 ist beispielhaft ein als Verbindungsbereich dienender Abschnitt der Ränder 3, 9 bezeichnet (siehe Fig. 5).

Die Unterseite des Randes 3 des Oberteils 1 ist im Verbindungsbereich 17 plan ausgebildet.

Insbesondere sind die Aufreißlasche 10 und die zur Schwächung dienenden umlaufenden Nuten 11, 12 weiter innen angeordnet als der Verbindungsbereich 17, so dass durch Betätigen der Aufreißlasche 10 die Verbindung von Oberteil 1 und Unterteil 5 gelöst werden kann.

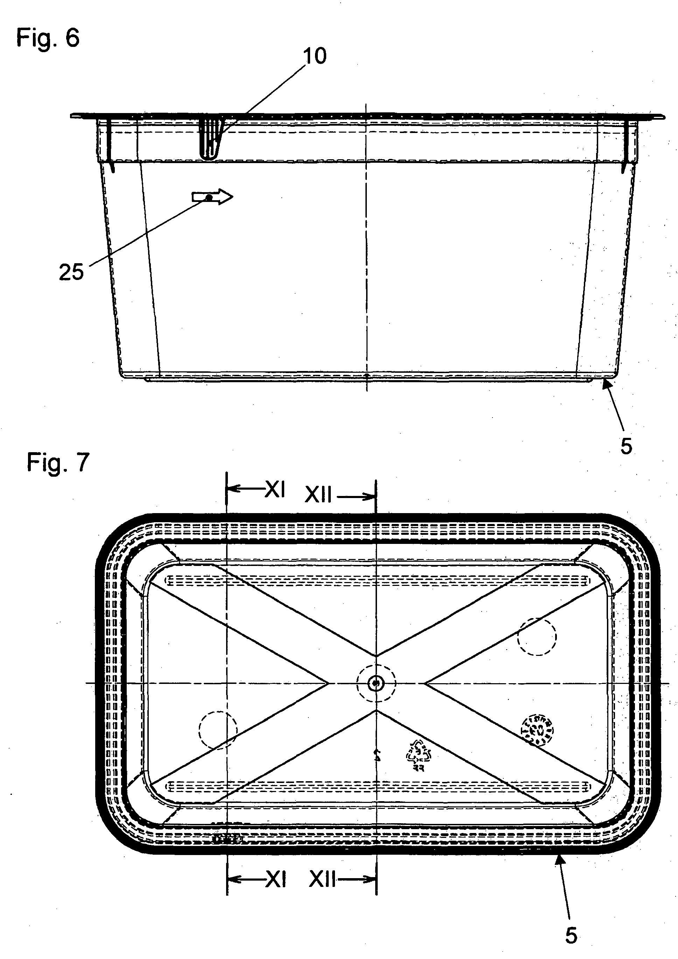

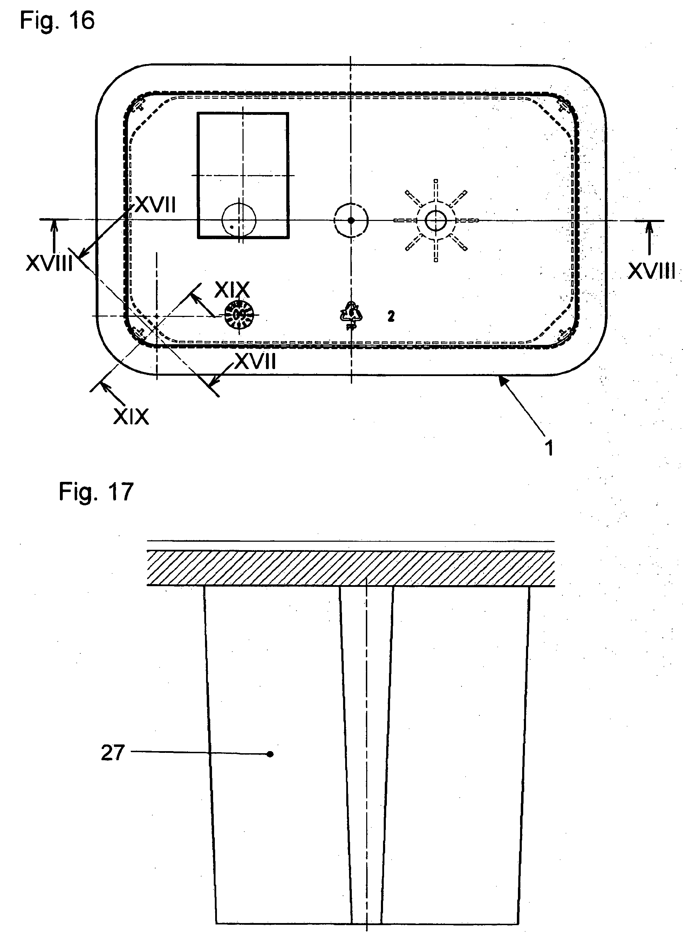

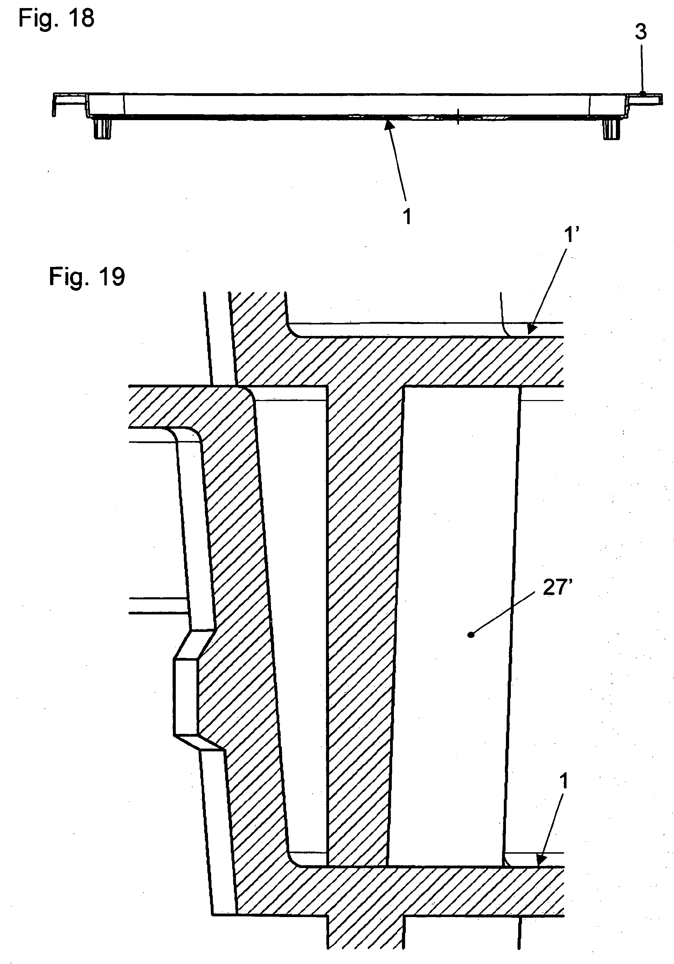

Bei den zweiten Ausführungsformen gemäß den Fig. 6 bis 19 sowie Fig. 21 und Fig. 22 sind gleiche oder funktional gleiche Teile mit gleichen Bezugszeichen versehen wie bei den Fig. 1 bis 5. Die zweiten Ausführungsformen unterscheiden sich nicht wesentlich von den ersten Ausführungsformen.

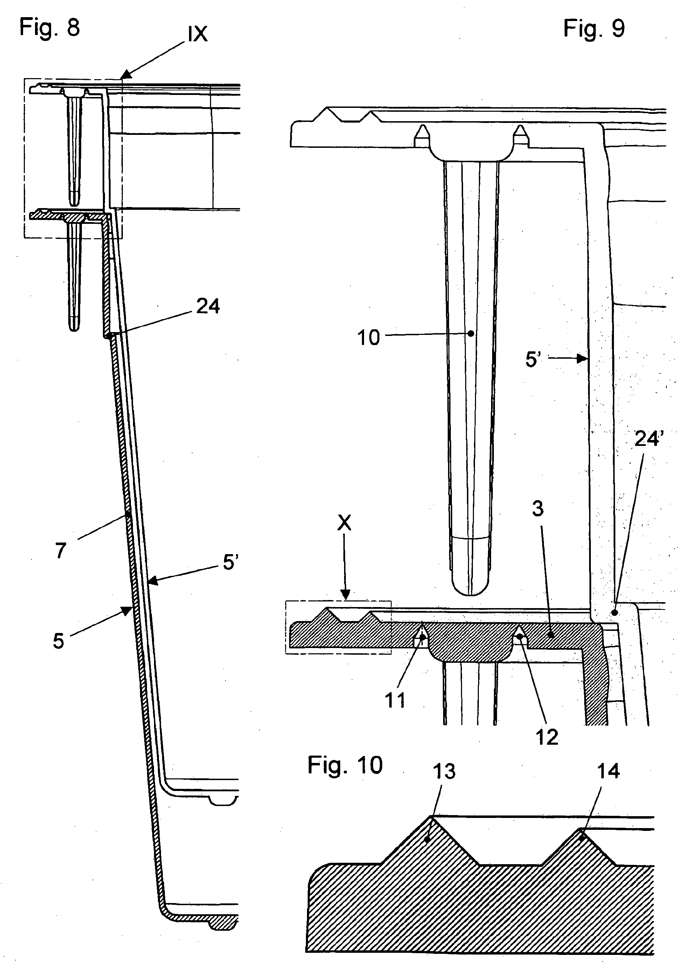

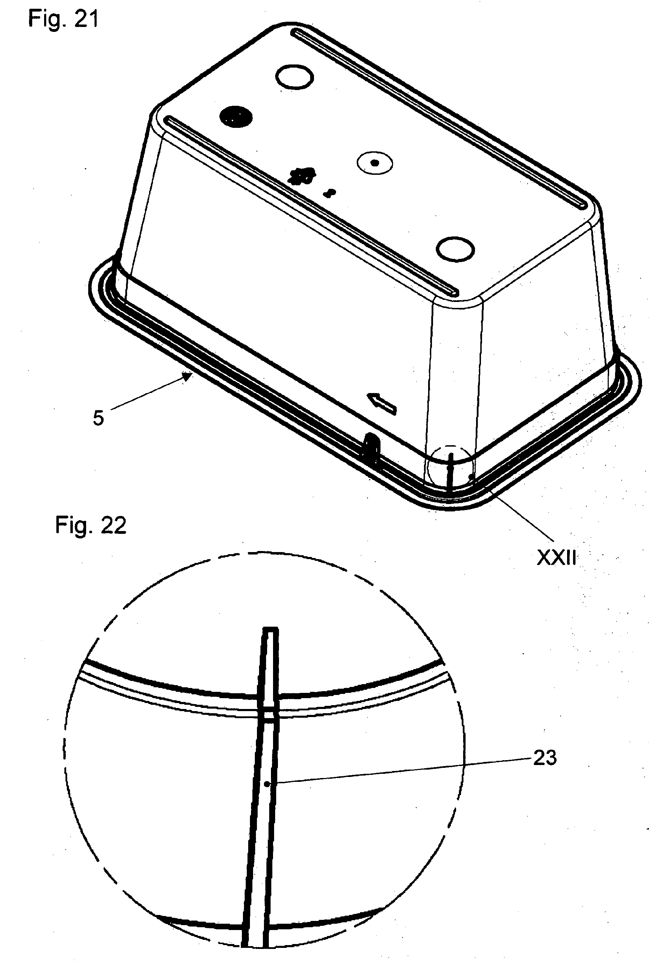

Die zweite Ausführungsform des Unterteils 5 gemäß den Fig. 6 bis 12 sowie Fig. 21 und Fig. 22 weist in jeder der Ecken eine Stapelrippe 23 auf, die von einem oberen Bereich der Seitenwände 7 nach außen ragt (siehe Fig. 22). Weiterhin weist jede der Seitenwände 7 eine von dem oberen Rand beabstandete Stufe 24 auf, ab der der untere Teil der Seitenwand 7 etwas zurückspringt. Die Stufe 24' eines oberen Unterteils 5' kann auf dem Rand 3 eines unteren Unterteils 5 aufliegen (siehe Fig. 9).

Die Stapelrippen 23 weisen eine größere Länge auf als die Aufreißlasche 10 (siehe dazu Fig. 21). Dadurch können beim Ineinanderstapeln die Aufreißlaschen 10 nicht beschädigt werden.

Durch die Stufen 24 und die Stapelrippen 23 können die Unterteile 5, 5' so ineinander gestapelt werden, dass sie einfach wieder auseinander gezogen werden können. Das Vorsehen der Stufen 24 ermöglicht eine etwas stärkere Neigung der Seitenwände 7 von beispielsweise 5°, ohne dass durch diese Neigung das Volumen des Unterteils 5 zu stark verkleinert wird. Durch diese Neigung wird das Entstapeln vereinfacht.

Fig. 6 verdeutlicht durch den Pfeil 25 die Aufreißrichtung, in die die Lasche 10 bewegt werden muss, um die Verbindung zwischen dem Oberteil 1 und dem Unterteil 5 zu lösen.

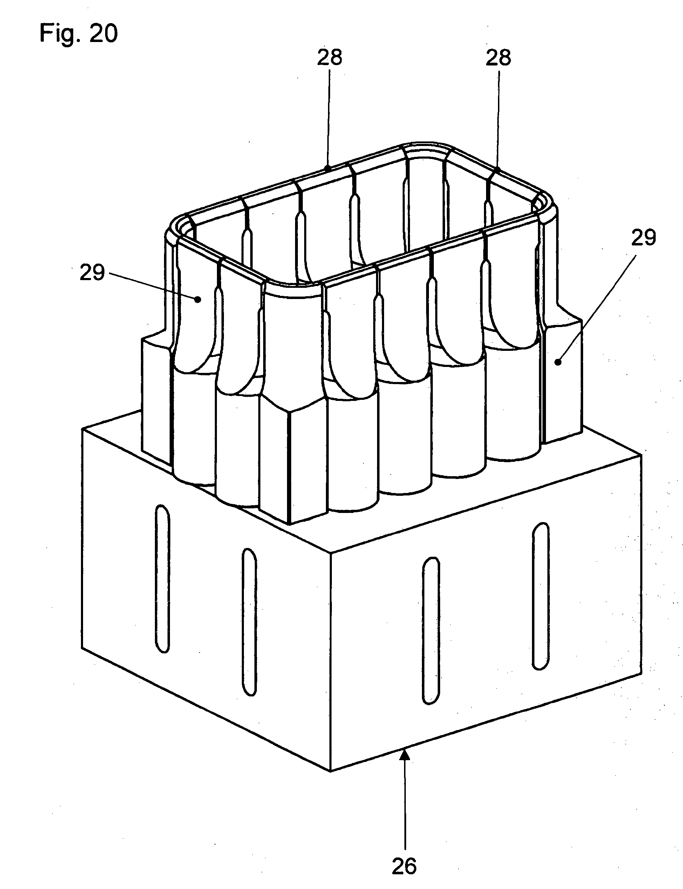

Fig. 10 zeigt, dass der weiter außen angeordnete Vorsprung 13 höher ist als der weiter innen angeordnete Vorsprung 14. Dabei wird beispielsweise mittels der in Fig. 20 abgebildeten Ultraschall-Sonotrode 26 der äußere Vorsprung 13 und der korrespondierende Bereich des Randes 3 des Oberteils 1 aufgeschmolzen, so dass dieses aufgeschmolzene Material besonders zu einer dichten Schweißverbindung beiträgt. Die Verkleinerung des inneren Vorsprungs 14 gegenüber dem äußeren Vorsprung 13 verhindert, dass aufgeschmolzenes Material in den im Bereich der Nuten 11, 12 angeordneten Sollbruchbereich gelangt und diesen verstärkt und damit unter Umständen unbrauchbar macht.

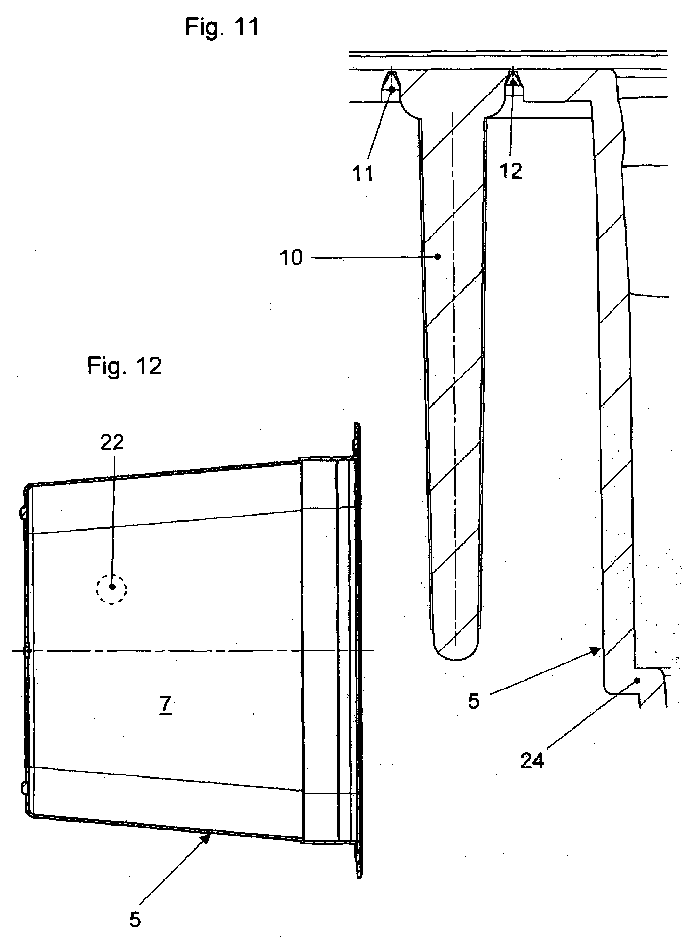

Das Unterteil 5 kann in einer seiner Seitenwände 7 eine Öffnung 22 aufweisen, die wie die Öffnung 4 des Oberteils 1 mit einem Filter versehen sein kann (in Fig. 12 ist diese Öffnung 22 gestrichelt angedeutet). Dabei kann die Öffnung 22 insbesondere in der unteren Hälfte der Seitenwand 7 angeordnet sein. Insbesondere ist die Öffnung 22 so tief wie möglich, jedoch oberhalb der typischen Einfüllhöhe des Nährbodens angeordnet. Dadurch kann gewährleistet werden, dass durch die Öffnung 22 CO₂ aus dem Behälter austreten kann.

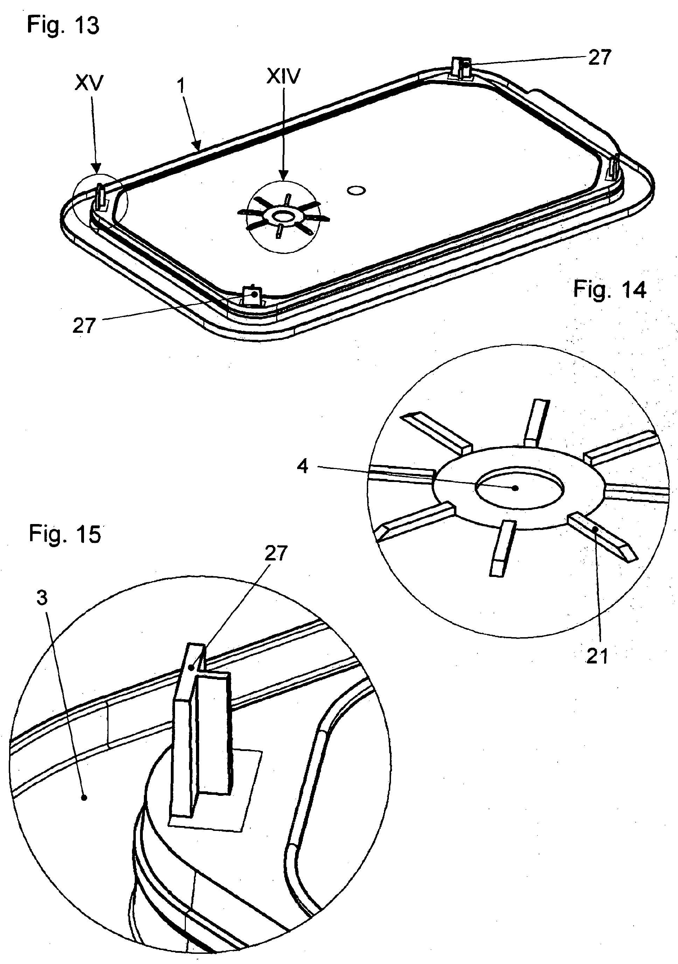

Bei der zweiten Ausführungsform des Oberteils 1 gemäß den Fig. 13 bis 19 ist die Unterseite des Randes 3 im Verbindungsbereich 17 nicht mit Nuten zu versehen, sondern plan ausgebildet (siehe beispielsweise Fig. 15).

Die zweite Ausführungsform des Oberteils 1 gemäß den Fig. 13 bis 19 weist weiterhin in jeder Ecke einen, beispielsweise T-förmigen, Stapelfuß 27 auf. Die Stapelfuß 27' eines oberen Oberteils 1' kann auf der Oberseite eines unteren Oberteils 1 aufstehen, so dass dadurch das Entstapeln der Oberteile 1, 1' besser ermöglicht wird, insbesondere bei einer geringen Schräge des Randbereichs.

Die Ränder 3, 9 des Oberteils 1 und des Unterteils 5 beziehungsweise der an ihnen angeordnete Verbindungsbereich 17 können in Umfangsrichtung beispielsweise eine Länge zwischen 50 cm und 55 cm aufweisen. Um einen derartig großen Verbindungsbereich durch Ultraschallschweißen in einem einzigen Arbeitsschritt zu erstellen, wird eine Ultraschall-Sonotrode 26 gemäß Fig. 20 verwendet.

Zur Durchführung des erfindungsgemäßen Verfahrens wird die Ultraschall-Sonotrode 26 an die Oberseite des Randes 3 eines Oberteils 1 angedrückt, während der Rand 3 auf dem Rand 9 eines Unterteils 5 aufliegt. Dabei passt der Umfang des Ultraschall abgebenden Endes 28 (oben in Fig. 20) der Sonotrode 26 genau zu dem Umfang der Ränder 3, 9. Während des Andrückens der Ultraschall-Sonotrode 26 an die Oberseite des Randes 3 wird die Ultraschall-Sonotrode 26 so betrieben, dass die Oberseite des Randes 3 mit Ultraschall beaufschlagt wird.

Die Ultraschall-Sonotrode 26 weist einzelne Segmente 29 auf, die in Umfangsrichtung beabstandet zueinander sind. Im Schweißbereich, der in Fig. 20 dem oberen Ende 28 der Ultraschall-Sonotrode 26 entspricht, beträgt der Abstand der einzelnen Segmente 29 zueinander etwa 1 mm. Etwas weiter unten ist der Abstand der einzelnen Segmente 29 zueinander etwas größer, so dass die Breite der einzelnen Segmente 29 sich in Richtung auf den Schweißbereich vergrößert. Dadurch wird erreicht, dass auch im Übergangsbereich von einem Segment 29 zum benachbarten Segment 29 ein gutes Schweißergebnis erzielt wird.

## Patentansprüche

1. Behälter für die Aufnahme von Gegenständen, umfassend
- ein Unterteil (5) mit einem Boden (6) sowie mit einer Öffnung (18) und einem über die Öffnung (18) zugänglichen Aufnahmeraum (8) für Gegenstände, wobei das Unterteil (5) einen zumindest teilweise umlaufenden Rand (9) aufweist, der sich parallel zu dem Boden (6) erstreckt,
- ein Oberteil (1), das eine Deckelfläche (2) und einen zumindest teilweise umlaufenden Rand (3, 9) aufweist, der sich parallel zu der Deckelfläche (2) erstreckt, wobei das Oberteil (1) mit dem Unterteil (5) über mindestens einen Verbindungsbereich (17) verbindbar ist, wobei die Ränder (3, 9) von Oberteil (1) und Unterteil (5) im verbundenen Zustand zumindest teilweise aneinander anliegen, wobei die Verbindung zwischen Oberteil (1) und Unterteil (5) durch Verbindung der Ränder (3, 9) miteinander realisiert ist und wobei der mindestens eine Verbindungsbereich (17) derart gestaltet ist, dass der Aufnahmeraum (8) dicht verschlossen, insbesondere keimfrei verschlossen ist, sowie
- Mittel zum Lösen der Verbindung zwischen Oberteil (1) und Unterteil (5), die zwischen der Öffnung (18) und dem mindestens einen Verbindungsbereich (17) angeordnet sind und einen Sollbruchbereich an dem Unterteil (5) oder an dem Oberteil (1) umfassen, der zwischen der Öffnung und dem mindestens einen Verbindungsbereich (17) angeordnet ist,
**dadurch gekennzeichnet, dass** die Mittel zum Lösen der Verbindung mindestens eine Aufreißlasche (10) umfassen, die benachbart zu dem mindestens einen Sollbruchbereich an dem Unterteil (5) oder an dem Oberteil (1) angeordnet ist.

2. Behälter nach Anspruch 1, **dadurch gekennzeichnet, dass** an dem Rand (3, 9) des Oberteils (1) und/oder des Unterteils (5) mindestens ein, vorzugsweise zwei, zumindest teilweise umlaufende, auf den jeweils anderen Rand (3, 9) hingerichtete Vorsprünge (13, 14) vorgesehen sind, von denen insbesondere der hinsichtlich der Öffnung (18) weiter außen liegende Vorsprung (13) höher ist als der hinsichtlich der Öffnung (18) weiter innen liegende Vorsprung (14).

3. Behälter nach einem der Ansprüche 1 oder 2, **dadurch gekennzeichnet, dass** der mindestens eine Verbindungsbereich (17) an einem der Ränder (3, 9) oder an beiden Rändern (3, 9) von Oberteil (1) und Unterteil (5) angeordnet ist.

4. Behälter nach einem der Ansprüche 1 bis 3, **dadurch gekennzeichnet, dass** der mindestens eine Verbindungsbereich (17) zumindest teilweise mit einem der Ränder (3, 9) oder beiden Rändern (3, 9) umläuft.

5. Behälter nach einem der Ansprüche 1 bis 4, **dadurch gekennzeichnet, dass** die Verbindung zwischen Oberteil (1) und Unterteil (5) als Ultraschallschweißverbindung oder als Laserschweißverbindung oder als Heißprägeverbindung oder als Klebeverbindung ausgebildet ist.

6. Behälter nach einem der Ansprüche 1 bis 5, **dadurch gekennzeichnet, dass** der Sollbruchbereich mindestens eine, vorzugsweise zwei, zumindest teilweise umlaufende Nuten (11, 12) umfasst.

7. Behälter nach einem der Ansprüche 1 bis 6, **dadurch gekennzeichnet, dass** die mindestens eine Aufreißlasche (10) senkrecht von dem Unterteil (5) oder dem Oberteil (1) wegragt und insbesondere im Querschnitt winkelförmig, wie beispielsweise L-förmig oder V-förmig ausgebildet ist.

8. Behälter nach einem der Ansprüche 1 bis 7, **dadurch gekennzeichnet, dass** das Oberteil (1) und/oder das Unterteil (5) eine Öffnung (4, 22) aufweisen, die mit einem Filter versehen ist oder versehen werden kann.

9. Behälter nach Anspruch 8, **dadurch gekennzeichnet, dass** die Öffnung (22) in Gebrauchsstellung beabstandet von dem oberen Rand (9) des Unterteils in einer der Seitenwände (7) des Unterteils (5) angeordnet ist.

10. Verfahren zur Herstellung einer Verbindung zwischen einem Unterteil (5) und einem Oberteil (1) eines Behälters für die Aufnahme von Gegenständen nach einem der Ansprüche 1 bis 9, wobei das Unterteil (5) eine Öffnung (18) und einen über die Öffnung (18) zugänglichen Aufnahmeraum (8) für Gegenstände umfasst, und wobei das Oberteil (1) mit dem Unterteil (5) über mindestens einen Verbindungsbereich (17) derart verbindbar ist, dass der Aufnahmeraum (8) dicht verschlossen ist, wobei der Behälter Mittel zum Lösen der Verbindung zwischen Oberteil (1) und Unterteil (5) umfasst, die zwischen der Öffnung (18) und dem mindestens einen Verbindungsbereich (17) angeordnet sind und einen Sollbruchbereich an dem Unterteil (5) oder an dem Oberteil (1) umfassen, der zwischen der Öffnung und dem mindestens einen Verbindungsbereich (17) angeordnet ist, wobei die Mittel zum Lösen der Verbindung mindestens eine Aufreißlasche (10) umfassen, die benachbart zu dem mindestens einen Sollbruchbereich an dem Unterteil (5) oder an dem Oberteil (1) angeordnet ist, und wobei das Oberteil (1) mit dem Unterteil (5) in dem mindestens einen Verbindungsbereich (17) mit Ultraschall verschweißt wird **dadurch gekennzeichnet, dass** für das Verschweißen eine Ultraschall-Sonotrode (26) verwendet wird, die eine Mehrzahl von Segmenten (29) aufweist, die zumindest abschnittsweise voneinander beabstandet sind, wobei die Breite der Segmente (29) sich in Richtung auf den Schweißbereich vergrößert.

## Claims

1. Container for receiving objects, comprising
- a lower part (5) with a base (6) and with an opening (18) and a receiving space (8), which is accessible via the opening (18), for objects, wherein the lower part (5) has an at least partially encircling edge (9), which extends parallel to the base (6),
- an upper part (1), which has a cover surface (2) and an at least partially encircling edge (3, 9), which extends parallel to the cover surface (2), wherein the upper part (1) can be connected to the lower part (5) via at least one connecting region (17), wherein the edges (3, 9) of the upper part (1) and lower part (5) are at least partially in contact with each other in the connected state, wherein the connection between the upper part (1) and lower part (5) is realized by connecting the edges (3, 9) to each other, and wherein the at least one connecting region (17) is designed in such a manner that the receiving space (8) is tightly closed, in particular is closed in a germ-free manner, and
- means for releasing the connection between the upper part (1) and lower part (5), which are arranged between the opening (18) and the at least one connecting region (17) and comprise a predetermined breaking region on the lower part (5) or on the upper part (1), said predetermined breaking region being arranged between the opening and the at least one connecting region (17),
**characterized in that** the means for releasing the connection comprise at least one tear-open tab (10) which is arranged adjacent to the at least one predetermined breaking region on the lower part (5) or on the upper part (1).

2. Container according to Claim 1, **characterized in that** at least one, preferably two, at least partially encircling projections (13, 14) are provided on the edge (3, 9) of the upper part (1) and/or of the lower part (5) and are directed toward the other edge (3, 9) in each case, and of which in particular the projection (13) which is further outward with respect to the opening (18) is higher than the projection (14) which is further inward with respect to the opening (18).

3. Container according to either of Claims 1 and 2, **characterized in that** the at least one connecting region (17) is arranged on one of the edges (3, 9) or on both edges (3, 9) of the upper part (1) and lower part (5).

4. Container according to one of Claims 1 to 3, **characterized in that** the at least one connecting region (17) is at least partially encircling together with one of the edges (3, 9) or both edges (3, 9).

5. Container according to one of Claims 1 to 4, **characterized in that** the connection between the upper part (1) and lower part (5) is designed as an ultrasonic welding connection or as a laser welding connection or as a hot embossed connection or as an adhesive bonded connection.

6. Container according to one of Claims 1 to 5, **characterized in that** the predetermined breaking region comprises at least one, preferably two, at least partially encircling grooves (11, 12).

7. Container according to one of Claims 1 to 6, **characterized in that** the at least one tear-open tab (10) projects away perpendicularly from the lower part (5) or the upper part (1) and in particular is of angled design in cross section, such as, for example, is of L-shaped or V-shaped design.

8. Container according to one of Claims 1 to 7, **characterized in that** the upper part (1) and/or the lower part (5) have/has an opening (4, 22) which is provided or can be provided with a filter.

9. Container according to Claim 8, **characterized in that** in the use position, the opening (22) is arranged in one of the side walls (7) of the lower part (5) at a distance from the upper edge (9) of the lower part.

10. Method for producing a connection between a lower part (5) and an upper part (1) of a container for receiving objects according to one of Claims 1 to 9, wherein the lower part (5) comprises an opening (18) and a receiving space (8), which is accessible via the opening (18), for objects, and wherein the upper part (1) can be connected to the lower part (5) via at least one connecting region (17) in such a manner that the receiving space (8) is tightly closed, wherein the container comprises means for releasing the connection between the upper part (1) and lower part (5), which are arranged between the opening (18) and the at least one connecting region (17) and comprise a predetermined breaking region on the lower part (5) or on the upper part (1), said predetermined breaking region being arranged between the opening and the at least one connecting region (17), wherein the means for releasing the connection comprise at least one tear-open tab (10) which is arranged adjacent to the at least one predetermined breaking region on the lower part (5) or on the upper part (1), and wherein by ultrasound the upper part (1) is welded to the lower part (5) in the at least one connecting region (17), **characterized in that** for the welding use is made of an ultrasonic sonotrode (26) which has a plurality of segments (29), at least sections of which are spaced apart from one another, wherein the width of the segments (29) increases in the direction of the weld region.

## Revendications

1. Récipient pour recevoir des objets, comprenant :
- une partie inférieure (5) avec un fond (6) ainsi qu'une ouverture (18) et un espace de réception (8) pour des objets, accessible par le biais de l'ouverture (18), la partie inférieure (5) présentant un bord (9) s'étendant au moins en partie sur la périphérie, qui s'étend parallèlement au fond (6),
- une partie supérieure (1) qui présente une surface de couvercle (2) et un bord (3, 9) s'étendant au moins en partie sur la périphérie, qui s'étend parallèlement à la surface de couvercle (2), la partie supérieure (1) pouvant être connectée à la partie inférieure (5) par le biais d'au moins une région de connexion (17), les bords (3, 9) de la partie supérieure (1) et de la partie inférieure (5) s'appliquant au moins en partie l'un contre l'autre dans l'état assemblé, la connexion entre la partie supérieure (1) et la partie inférieure (5) étant réalisée par la connexion des bords (3, 9) l'un avec l'autre, et l'au moins une région de connexion (7) étant configurée de telle sorte que l'espace de réception (8) soit fermé hermétiquement, en particulier soit fermé de manière stérile, et
- des moyens pour desserrer la connexion entre la partie supérieure (1) et la partie inférieure (5), qui sont disposés entre l'ouverture (18) et l'au moins une région de connexion (17) et qui comprennent une région destinée à la rupture au niveau de la partie inférieure (5) ou de la partie supérieure (1), laquelle est disposée entre l'ouverture et l'au moins une région de connexion (17),
**caractérisé en ce que** les moyens pour desserrer la connexion comprennent au moins une patte de déchirure (10) qui est disposée à côté de l'au moins une région destinée à la rupture au niveau de la partie inférieure (5) ou de la partie supérieure (1).

2. Récipient selon la revendication 1, **caractérisé en ce qu'**au niveau du bord (3, 9) de la partie supérieure (1) et/ou de la partie inférieure (5) sont prévues au moins une, de préférence deux, saillies (13, 14) au moins en partie périphériques, orientées vers l'autre bord respectif (3, 9), dont notamment la saillie (13) située le plus à l'extérieur par rapport à l'ouverture (18) est plus haute que la saillie (14) située le plus à l'intérieur par rapport à l'ouverture (18).

3. Récipient selon l'une quelconque des revendications 1 ou 2, **caractérisé en ce que** l'au moins une région de connexion (17) est disposée au niveau de l'un des bords (3, 9) ou au niveau des deux bords (3, 9) de la partie supérieure (1) et de la partie inférieure (5).

4. Récipient selon l'une quelconque des revendications 1 à 3, **caractérisé en ce que** l'au moins une région de connexion (17) s'étend au moins en partie sur la périphérie avec l'un des bords (3, 9) ou avec les deux bords (3, 9).

5. Récipient selon l'une quelconque des revendications 1 à 4, **caractérisé en ce que** la connexion entre la partie supérieure (1) et la partie inférieure (5) est réalisée sous forme de connexion par soudage par ultrasons ou sous forme de connexion par soudage au laser ou sous forme de connexion par marquage à chaud ou sous forme de connexion par collage.

6. Récipient selon l'une quelconque des revendications 1 à 5, **caractérisé en ce que** la région destinée à la rupture comprend au moins une, de préférence au moins deux, rainures au moins en partie périphériques (11, 12).

7. Récipient selon l'une quelconque des revendications 1 à 6, **caractérisé en ce que** l'au moins une patte de déchirure (10) fait saillie perpendiculairement depuis la partie inférieure (5) ou la partie supérieure (1) et est réalisée notamment avec une section transversale de forme coudée, par exemple en forme de L ou en forme de V.

8. Récipient selon l'une quelconque des revendications 1 à 7, **caractérisé en ce que** la partie supérieure (1) et/ou la partie inférieure (5) présente (nt) une ouverture (4, 22) qui est pourvue ou qui peut être pourvue d'un filtre.

9. Récipient selon la revendication 8, **caractérisé en ce que** l'ouverture (22), dans la position d'utilisation, est disposée à distance du bord supérieur (9) de la partie inférieure dans l'une des parois latérales (7) de la partie inférieure (5).

10. Procédé de fabrication d'une connexion entre une partie inférieure (5) et une partie supérieure (1) d'un récipient pour recevoir des objets selon l'une quelconque des revendications 1 à 9, la partie inférieure (5) comprenant une ouverture (18) et un espace de réception (8) pour des objets accessibles par le biais de l'ouverture (18), et la partie supérieure (1) pouvant être connectée à la partie inférieure (5) par le biais d'au moins une région de connexion (17), de telle sorte que l'espace de réception (8) soit fermé hermétiquement, le récipient comprenant des moyens pour desserrer la connexion entre la partie supérieure (1) et la partie inférieure (5), lesquels sont disposés entre l'ouverture (18) et l'au moins une région de connexion (17), et comprennent une région destinée à la rupture au niveau de la partie inférieure (5) ou de la partie supérieure (1), laquelle est disposée entre l'ouverture et l'au moins une région de connexion (17), les moyens pour desserrer la connexion comprenant au moins une patte de déchirure (10) qui est disposée à côté de l'au moins une région destinée à la rupture au niveau de la partie inférieure (5) ou au niveau de la partie supérieure (1), et la partie supérieure (1) étant soudée par ultrasons à la partie inférieure (5) dans l'au moins une région de connexion (17), **caractérisé en ce que** pour le soudage, on utilise une sonotrode à ultrasons (26) qui présente une pluralité de segments (29) qui sont espacés au moins en partie les uns des autres, la largeur des segments (29) augmentant dans la direction de la région de soudage.
